# EUROPÄISCHE PATENTANMELDUNG

(11) **EP 0 665 017 A1**
(43) Veröffentlichungstag der Anmeldung: **02.08.1995**
(21) Anmeldenummer: 93121109.8
(22) Anmeldetag: 30.12.1993
(51) Int. Cl.: A61K 35/78

(54) **Arzneimittel für die Tumorprophylaxe und -behandlung**

(71) Anmelder: Henne, Kurt, D-72510 Stetten am kalten Markt (DE)
(72) Erfinder: Peizhong, Lin, Prof. Dr., CN-100083 Bejing (CN)
(74) Vertreter: Kodron, Rudolf S., Dipl.-Ing.

(57) **Zusammenfassung**

Es wird angestrebt, auf naturheilkundlichem Wege die inneren einer Krebserkrankung entgegenwirkenden Faktoren im Körper zu stärken, welche den äußeren kanzerogenen Faktoren entgegenwirken, indem das Immunsystem gestärkt, eine Zellentgiftung gefördert und neu gebildete Krebszellen durch körpereigene Killerzellen und Antikörper zerstört werden.

Erreicht wird dies durch ein naturheilkundliches Präparat aus chinesischen Kräuterpflanzen in der Arzneiform eines Dragees von z.B. 3,333̇g Gewicht und mit Einzelanteilen von

| | |
|---|---|
| Sophora tonkinensis Gapnep | 42,0 mg |
| Sonchus brachyotus DC. | 42,0 mg |
| Dictamnus dasycarpus Turcz. | 21,0 mg |
| Prunella vulgaris L. | 42,0 mg |
| Polygonum bistorta L. | 42,0 mg |
| Dioscorsa bulbifera L. | 10,0 mg. |

## Beschreibung

Die Erfindung betrifft ein Arzneimittel für die Tumorprophylaxe und -behandlung.

Es gibt viele unterschiedliche Arten von Krebserkrankungen, die alle nicht auf einer einheitlichen Ursache beruhen, sondern auf zahlreichen kanzerogenen Faktoren (Risikofaktoren) äußerer und innerer Art.

Zu den äußeren kanzerogenen Faktoren zählen diverse Chemikalien (z.B. polyzyklische aromatische Kohlenwasserstoffe), physikalische Einwirkungen, beispielsweise energiereiche ionisierende Strahlen, und Tumorviren.

Der Eintritt und das Ausmaß der Zellschädigung bei einer Krebserkrankung durch äußere Einwirkungen ist jedoch auch von inneren Reaktionen im Körper abhängig. Ein schwaches oder geschädigtes Immunsystem der Körperzellen läßt das Risiko einer Krebserkrankung um ein Vielfaches steigen. Als weiterer innerer Faktor kommt die Wirkung von Hormonen in Betracht.

Der Erfindung liegt die Aufgabe zugrunde, auf naturheilkundlichem Wege die inneren Faktoren zu stärken, welche den äußeren kanzerogenen Faktoren entgegenwirken, indem das Immunsystem gestärkt, eine Zellentgiftung gefördert und neu gebildete Krebszellen durch körpereigene Killerzellen und Antikörper zerstört werden.

Gelöst wird diese Aufgabe nach der Erfindung durch ein naturheilkundliches Präparat aus chinesischen Kräuterpflanzen in der Arzneiform eines Dragees von z.B. 3,333̇g Gewicht und mit Einzelanteilen von

| | |
|---|---|
| Sophora tonkinensis Gapnep | 42,0 mg |
| Sonchus brachyotus DC. | 42,0 mg |
| Dictamnus dasycarpus Turcz. | 21,0 mg |
| Prunella vulgaris L. | 42,0 mg |
| Polygonum bistorta L. | 42,0 mg |
| Dioscorsa bulbifera L. | 10,0 mg |

Dieses Präparat erhielt im November 1992 als T.C.M.-Präparat (T.C.M. = Traditional Chinese Medicine) "Zeng Sheng Ping Pian" die staatliche Arzneimittelzulassung in China.

Seit 1983 wurde das Mittel in China (Provinz Henan) im Rahmen des Sechsten und Siebten Fünfjahresplans durch die Chinesische Akademie der Medizinwissenschaften an 5.924 Personen mit früh erkanntem Krebs im Mund-Speisenrohren-Verdauungstrakt drei bis fünf Jahre lang regelmäßig verabreicht.

Es wurde eine 50%ige Herabsetzung bzw. Heilung der Krebserkrankungen erzielt.

Diese naturheilkundliche Krebstherapie ist im Gegensatz zu den rabiaten schulmedizinischen Chirurgie-, Strahlen- und Chemo-Therapiemethoden vollkommen frei von schädlichen Nebenwirkungen, ist lebensqualitätssteigernd und -im Bedarfsfall- palliativ.

## Patentansprüche

1. Arzneimittel für die Tumorprophylaxe und -behandlung gekennzeichnet durch
- ein naturheilkundliches Präparat aus chinesischen Kräuterpflanzen
- in der Arzneiform eines Dragées von z.B. 3,333̇g Gewicht und
- mit Einzelanteilen von
| | |
|---|---|
| Sophora tonkinensis Grapnep | 42,0 mg |
| Sonchus brachyotus DC. | 42,0 mg |
| Dictamnus dasycarpus Turcz. | 21,0 mg |
| Prunella vulgaris L. | 42,0 mg |
| Polygonum bistorta L. | 42,0 mg |
| Dioscorsa bulbifera L. | 10,0 mg. |
